# EUROPEAN PATENT APPLICATION

(11) **EP 1 473 033 A1**
(43) Date of publication of application: **03.11.2004**
(21) Application number: 03700558.4
(22) Date of filing: 15.01.2003
(51) Int. Cl.: A61K 31/192, A61K 47/38, A61P 3/06, A61P 3/10

(54) **MEDICINAL COMPOSITION CONTAINING 2,2-DICHLORO-12-(4-CHLOROPHENYL)DODECANOIC ACID**

(30) Priority: 16.01.2002 JP 2002007022
(71) Applicant: Kowa Company Ltd., Nagoya-shi Aichi-ken, 460-8625 (JP)
(72) Inventor: KOBAYASHI, Shin-ichiro, Fuji-shi, Shizuoka 417-0845 (JP); TAKANO, Niichiro, Fuji-shi, Shizuoka 416-0921 (JP); KAWASHIMA, Hiroyuki, Fuji-shi, Shizuoka 417-0014 (JP); SHINODA, Yasuo, Shizuoka-shi, Shizuoka 420-0867 (JP); INAGI, Toshio, Mishima-shi, Shizuoka 411-0038 (JP)
(74) Representative: Sternagel, Fleischer, Godemeyer & Partner Patentanwälte
(86) International application number: PCT/JP2003/000251
(87) International publication number: WO 2003/059337

(57) **Abstract**

A highly stable pharmaceutical composition which comprises a mixture comprising a substance selected from the group consisting of 2,2-dichloro-12-(4-chlorophenyl)-dodecanoic acid, salts thereof, and esters thereof, and croscarmellose sodium.

## Description

### Technical Field

The present invention relates to a highly stable pharmaceutical composition comprising 2,2-dichloro-12-(4-chlorophenyl)-dodecanoic acid.

### Background Art

2,2-Dichloro-12-(4-chlorophenyl)-dodecanoic acid has valuable pharmacological properties, such as normalization of a high blood sugar level without a risk of hypoglycemia as well as reduction of triglyceride, cholesterol, and fibrinogen, and thus is promising as a medicament for treatment of diabetes mellitus (Japanese Patent Publication [Kohyo] No.10-510515/1998). However, the aforementioned compound decomposes chronologically, which leads to a decrease of a content. Therefore, an improvement of stability in pharmaceutical preparations has been desired.

Examples of means to prevent unstabilization caused by moisture or oxygen include, in general, a method of application of a wax-coating to pharmaceuticals or mixing of corn starch which is capable of retaining moisture, and a method of preservation of pharmaceuticals under nitrogen substitution. However, when a wax-coating is applied to the aforementioned compound, a problem, retardation of dissolution of the active ingredient, may occur. When corn starch is admixed even at a sufficient amount to retain moisture, another problem arises, that said ingredient may fail to achieve a satisfactory effect and deteriorate formative property at compression. The preservation method with nitrogen substitution is also undesirable because of its poor stabilization effect and complicated manufacturing process. Therefore, development of a means to improve stability of the aforementioned compound has been desired.

### Disclosure of the Invention

An object of the present invention is to provide a highly stable pharmaceutical composition comprising 2,2-dichloro-12-(4-chlorophenyl)-dodecanoic acid.

The inventors of the present invention made intensive research to achieve the foregoing object. As a result, the inventors found that, when 2,2-dichloro-12-(4-chlorophenyl)-dodecanoic acid and croscarmellose sodium were mixed, stability of 2,2-dichloro-12-(4-chlorophenyl)-dodecanoic acid in the mixture was improved. The present invention was achieved on the basis of the above findings.

The present invention thus provides a pharmaceutical composition which comprises a mixture comprising a substance selected from the group consisting of 2,2-dichloro-12-(4-chlorophenyl)-dodecanoic acid, salts thereof, and esters thereof, and croscarmellose sodium.

According to a preferred embodiment of the present invention, the present invention provides the aforementioned pharmaceutical composition in which a mixing ratio of the substance selected from the group consisting of 2,2-dichloro-12-(4-chlorophenyl)-dodecanoic acid, salts thereof, and esters thereof, and croscarmellose sodium is from 10:1 to 1:20.

From another aspect, the present invention provides a stabilizer of a substance selected from the group consisting of 2,2-dichloro-12-(4-chlorophenyl)-dodecanoic acid, salts thereof, and esters thereof, which comprises croscarmellose sodium.

From further aspect, the present invention provides a method for stabilization of a substance selected from the group consisting of 2,2-dichloro-12-(4-chlorophenyl)-dodecanoic acid, salts thereof, and esters thereof, which comprises the step of preparing a mixture comprising a substance selected from the group consisting of 2,2-dichloro-12-(4-chlorophenyl)-dodecanoic acid, salts thereof, and esters thereof, and croscarmellose sodium.

### Best Mode for Carrying out the Invention

The pharmaceutical composition of the present invention comprises, as an active ingredient, a substance selected from the group consisting of 2,2-dichloro-12-(4-chlorophenyl)-dodecanoic acid, salts thereof, and esters thereof. The salts include salts of alkali metals, salts of alkaline earth metals, ammonium salt, salts of alkyl ammoniums or the like. Examples include, sodium salt, potassium salt, magnesium salt, calcium salt, tetramethyl ammonium salt or the like. Examples of the esters include esters with C1 to C6 aliphatic alcohols which are for example methyl ester, ethyl ester, propyl ester, butyl ester, isopropyl ester or the like. Among the aforementioned compounds, sodium salt of 2,2-dichloro-12-(4-chlorophenyl)-dodecanoic acid is particularly preferred.

2,2-Dichloro-12-(4-chlorophenyl)-dodecanoic acid can be prepared, for example, by the method described in Japanese Patent Publication [Kohyo] No.10-510515/1998. Salts and esters thereof can also be readily prepared by those skilled in the art with an ordinary method.

Croscarmellose sodium used in the present invention is a crosslinked polymer of carboxymethylcellulose sodium, and is commercially available as, for example, Ac-Di-Sol (Asahi Kasei Co., Ltd.) or the like. Croscarmellose sodium is widely used in the art as an excipient, a disintegrant, or a disintegrating adjuvant, and easily available to those in the art.

In the mixture comprising the substance selected from the group consisting of 2,2-dichloro-12-(4-chlorophenyl)-dodecanoic acid, salts thereof, and esters thereof, and croscarmellose sodium, a mixing ratio of the components is not particularly limited. For example, the ratio of the aforementioned substance and croscarmellose sodium is preferably from 10:1 to 1:20, and the most preferably from 2:1 to 1:15.

One or more pharmaceutical additives generally used for manufacture of pharmaceutical preparations may be further added to the pharmaceutical composition of the present invention in addition to the aforementioned ingredients, if required. The examples of such pharmaceutical additives include excipients, binders, disintegrants, lubricants or the like.

Examples of the excipients include lactose, saccharose, starch, microcrystalline cellulose, sucrose, mannitol, xylitol, hydrogenated oil, light anhydrous silicic acid, dibasic calcium phosphate or the like. An amount of the excipient may preferably be from 10 to 95% by weight, more preferably from 30 to 90% by weight, and the most preferably from 60 to 90% by weight based on the total weight of the composition.

Examples of the binder include methylcellulose, hydroxypropylcellulose, hydroxypropylmethylcellulose, pregelatinized starch, polyvinylpyrrolidone, polyvinylalcohol, gelatin, pullulan or the like. An amount of the binder may preferably be from 1 to 10% by weight, more preferably from 2 to 8% by weight, and the most preferably from 3 to 6% by weight based on the total weight of the composition.

Examples of the disintegrant include carmellose calcium, low-substituted hydroxypropylcellulose, corn starch, sodium carboxymethyl starch or the like. An amount of the disintegrant may preferably be from 2 to 25% by weight, more preferably from 3 to 15% by weight, and the most preferably from 4 to 10% by weight based on the total weight of the composition.

Examples of the lubricants include magnesium stearate, calcium stearate, talc or the like. An amount of the lubricants may preferably be from 0.01 to 5.0% by weight, more preferably from 0.1 to 2.0% by weight, and most preferably from 0.5 to 1.0% by weight based on the total weight of the composition.

When the aforementioned pharmaceutical additives are used, an amount of the substance selected from the group consisting of 2,2-dichloro-12-(4-chlorophenyl)-dodecanoic acid, salts thereof, and esters thereof may preferably be from 0.1 to 30% by weight, more preferably from 0.1 to 20% by weight, and the most preferably from 0.1 to 10% by weight based on the total weight of the composition. When the aforementioned pharmaceutical additives are used, an amount of the croscarmellose sodium may preferably be from 0.1 to 30% by weight, more preferably from 0.1 to 20% by weight, and the most preferably from 0.1 to 15% by weight based on the total weight of the composition.

The pharmaceutical composition of the present invention can be prepared by mixing a given amount of the substance selected from the group consisting of 2,2-dichloxo-12-(4-chlorophenyl)-dodecanoic acid, salts thereof, and esters thereof and croscarmellose sodium by using, for example, a V-blender or the like. For preparation of the aforementioned mixture, pharmaceutical additives such as excipients, disintegrants, binders or the like may be added to the mixture, if necessary. After an appropriate formulation process of the mixture is applied as required, such as pulverization or granulation, the pharmaceutical composition such as tablets or capsules may be prepared by suitable means available to those skilled in the art.

For preparation of the pharmaceutical compositions, the aforementioned mixture, per se, may be used. Alternatively, tablets or capsules may be manufactured by adding appropriate pharmaceutical additives to the mixture as required and by applying ordinary procedures available to those skilled in the art. For example, tablets can be prepared by adding pharmaceutical additives such as an excipient and a binder to the aforementioned mixture and then compressing with wet granulation (wet process) or direct compression (dry process). Capsules can be prepared by pulverization or granulation of aforementioned mixture and subsequently filling the resulting product in gelatin capsules, HPMC (hydroxypropylmethylcellulose) capsules or the like. Forms of the pharmaceutical composition of the present invention are not particularly limited so far that they are solid formulations, and may be any of powders, granules, chewables, film coated tablets, sugar coated tablets or the like, as well as tablets and capsules.

The mixture comprising the substance selected from the group consisting of 2,2-dichloro-12-(4-chlorophenyl)-dodecanoic acid, salts thereof, and esters thereof, and croscarmellose sodium may be either of the mixture by the wet process or the dry process. For example, when the wet process is employed, the mixture may preferably be a kneaded mass which is obtained by uniform kneading after the addition of 10 to 40% by weight of a binding liquid such as water, ethanol, isopropanol or the like to the aforementioned mixture. After the resulting kneaded mass is dried in vacuum, the resulting product may be sized, if necessary, and then mixed with magnesium stearate or the like to prepare tablets or capsules by the above processes.

The pharmaceutical composition of the present invention manufactured as described above is stable at a high temperature. For example, as for the tablets, a residual ratio of sodium 2,2-dichloro-12-(4-chlorophenyl)-dodecanoate after a two-week storage under a condition at 60°C is not less than 90%, and as for the capsules, a residual ratio of sodium 2,2-dichloro-12-(4-chlorophenyl)-dodecanoate after one-week storage under a condition at 60°C is not less than 85%.

The pharmaceutical composition of the present invention is useful as a medicament for therapeutic treatment of diabetes-related diseases. Generally, the medicament may be administered once a day or several times a day as divided portions normally in a daily dose of 1 to 600 mg as the weight of sodium 2,2-dichloro-12-(4-chlorophenyl)-dodecanoate.

### Examples

The present invention will be explained more specifically with reference to examples. However, the present invention is not limited to the following examples.

### Example 1

Sodium 2,2-dichloro-12-(4-chlorophenyl)-dodecanoate (10.0 g), lactose (1150.0 g), hydroxypropylcellulose (48.0 g), and croscarmellose sodium (120.0 g, tradename: Ac-Di-Sol, Asahi Kasei Co., Ltd.) were mixed for 10 minutes by using planetary mixer (Shinagawa-shiki-kongo-kakuhan-ki; Dalton 5DML-03-R), and then the mixture was added with 400.0 ml of purified water and kneaded. Then, the resulting kneaded mass was dried in vacuum for 4 hours under a condition at 60°C, sized by cutting mill (Speed Mill), mixed with 12.0 g of magnesium stearate by using a V-blender, and compressed by using tablet press (Kikusui rotary compressor 19TU) to obtain tablets (134.0 mg per tablet).

### Example 2

Sodium 2,2-dichloro-12-(4-chlorophenyl)-dodecanoate (10.0 g), lactose (1058.0 g), and croscarmellose sodium (120.0 g, trade name: Ac-Di-Sol, Asahi Kasei Co., Ltd.) were mixed for 10 minutes by using a V-blender. The mixture was then added with 12.0 g of magnesium stearate and mixing was further continued by using the V-blender. The mixture was compressed by using tablet press (Kikusui rotary compressor 19TU) to obtain tablets (120.0 mg per tablet).

### Example 3 to Example 5

Tablets were prepared as 80.0 mg per tablet in a similar manner to that of Example 2 with different contents of croscarmellose sodium.

### Comparative Example 1

Tablets were prepared as 134.0 mg per tablet in a similar manner to that of Example 1 by using low-substituted hydroxypropylcellulose, as also a disintegrant, instead of croscarmellose sodium.

### Comparative Example 2

Tablets obtained in Comparative Example 1 were placed in glass bottles, and air in the bottles was substituted by nitrogen gas.

### Comparative Example 3

Tablets were prepared as 134.0 mg per tablet in a similar manner to that of comparative example 1 with further addition of corn starch.

### Comparative tests and results

Each of the tablets obtained in Example 1 to 5 and Comparative Example 1 to 3 was placed in a glass bottle, and each bottle was tightly sealed and stored for 2 weeks under the condition at 60°C. Each content of sodium 2,2-dichloro-12-(4-ehlorophenyl)-dodecanoate was determined by HPLC measurement after the 2-week storage. The results are shown in Table 1.

**Table 1**

| | Example 1 | Example 2 | Example 3 | Example 4 | Example 5 | Comparative Example 1 | Comparative Example 2 | Comparative Example 3 |
|---|---|---|---|---|---|---|---|---|
| Sodium 2,2-dichloro-12-(4-chlorophenyl)-dodecanoate | 1.0 | 1.0 | 5.0 | 5.0 | 5.0 | 1.0 | 1.0 | 1.0 |
| Croscarmellose sodium | 12.0 | 12.0 | 4.0 | 8.0 | 12.0 | - | - | - |
| Low-substituted hydroxypropylcellulose | - | - | - | - | - | 12.0 | 12.0 | 12.0 |
| Corn starch | - | - | - | - | - | - | - | 12.0 |
| Lactose | 115.0 | 105.8 | 70.2 | 66.2 | 62.2 | 115.0 | 115.0 | 103.0 |
| Hydroxypropylcellulose | 4.8 | - | - | - | - | 4.8 | 4.8 | 4.8 |
| Magnesium stearate | 1.2 | 1.2 | 0.8 | 0.8 | 0.8 | 1.2 | 1.2 | 1.2 |
| Total(mg) | 134.0 | 120.0 | 80.0 | 80.0 | 80.0 | 134.0 | 134.0 | 134.0 |
| Residual ratio after storage at 60°C for 2weeks (%) | 93.7 | 93.9 | 96.3 | 97.2 | 97.8 | 84.9 | 88.3 | 87.8 |

Tablets of Comparative Example 1, where low-substituted hydroxypropylcellulose was used as a disintegrant instead of croscarmellose sodium, gave poor stability of sodium 2,2-dichloro-12-(4-chlorophenyl)-dodecanoate. Satisfactorily high stability was not achieved either by the storage under nitrogen substitution by eliminating oxygen (Comparative Example 2), or by the admixture of corn starch having moisture retaining property (Comparative Example 3).

In contrast, each of tablets of Example 1 to 5, where croscarmellose sodium was mixed, sodium 2,2-dichloro-12-(4-chlorophenyl)-dodecanoate was found to be stable after the storage for 2 weeks under a condition at 60°C.

### Example 6

Sodium 2,2-dichloro-12-(4-chlorophenyl)-dodecanoate (1.0 g), mannitol (211.5 g), talc (25.0 g), and croscarmellose sodium (12.5 g) were mixed by a V-blender for 10 minutes to obtain mixed powder. The resulting mixed powder (250.0 mg) was filled in HPMC capsule (size No.1) by using a capsule filler to obtain a capsule.

### Comparative Example 4

A capsule of 250.0 mg was prepared in a similar manner to that of Example 6 except that croscarmellose sodium was not mixed.

### Comparative Example 5

The capsule obtained in Comparative Example 4 was placed in a glass bottle and air in the bottle was substituted with nitrogen gas.

### Comparative Example 6

A capsule of 250.0 mg was prepared in a similar manner to that of Example 6 by using low-substituted hydroxypropylcellulose, as also a disintegrant, instead of croscarmellose sodium .

### Comparative Example 7

A capsule of 250.0 mg was prepared in a similar manner to that of Example 6 by using sodium carboxymethyl starch, as also a disintegrant, instead of croscarmellose sodium.

### Comparative tests and results

With a similar manner to the above comparative tests for the tablets, residual amounts of sodium 2,2-dichloro-12-(4-chlorophenyl)-dodecanoate were determined after storage of each of the capsules obtained in Example 6 and Comparative Examples 4 to 7 for a week under a condition at 60°C. The results are shown in Table 2.

**Table 2**

| | Example 6 | Comparative Example 4 | Comparative Example 5 | Comparative Example 6 | Comparative Example 7 |
|---|---|---|---|---|---|
| Sodium 2,2-dichloro-12-(4-chlorophenyl)-dodecanoate | 1.0 | 1.0 | 1.0 | 1.0 | 1.0 |
| Mannitol | 211.5 | 224.0 | 224.0 | 211.5 | 211.5 |
| Talc | 25.0 | 25.0 | 25.0 | 25.0 | 25.0 |
| Croscarmellose sodium | 12.5 | - | - | - | - |
| Low-substituted hydroxypropylcellulose | - | - | - | 12.5 | - |
| Sodium carboxymethyl starch | - | - | - | - | 12.5 |
| Total(mg) | 250.0 | 250.0 | 250.0 | 250.0 | 250.0 |
| Residual ratio after storage at 60°C for 1 week (%) | 88.5 | 70.1 | 73.5 | 67.7 | 57.6 |

Similar to the results of the tablets, the capsule of Example 6 mixed with croscarmellose sodium was stable after the storage for one week under a condition at 60°C compared with the other capsules of comparative examples.

### Industrial Applicability

The present invention provides a highly stable pharmaceutical composition comprising 2,2-dichloro-12-(4-chlorophenyl)-dodecanoic acid.

## Claims

1. A pharmaceutical composition which comprises a mixture comprising: a substance selected from the group consisting of 2,2-dichloro-12-(4-chlorophenyl)-dodecanoic acid, a salt thereof, and an ester thereof, and croscarmellose sodium.

2. The pharmaceutical composition according to claim 1 in which a mixing ratio of the substance selected from the group consisting of 2,2-dichloro-12-(4-chlorophenyl)-dodecanoic acid, the salt thereof, and the ester thereof, and the croscarmellose sodium is from 10:1 to 1:20.

3. A stabilizer for a substance selected from the group consisting of 2,2-dichloro-12-(4-chlorophenyl)-dodecanoic acid, a salt thereof, and an ester thereof, which comprises croscarmellose sodium.

4. A method for stabilization of a substance selected from the group consisting of 2,2-dichloro-12-(4-chlorophenyl)-dodecanoic acid, a salt thereof, and an ester thereof, which comprises the step of preparing a mixture comprising the substance selected from the group consisting of 2,2-dichloro-12-(4-chlorophenyl)-dodecanoic acid, the salt thereof, and the ester thereof, and croscarmellose sodium.
